# EUROPEAN PATENT SPECIFICATION

(11) **EP 1 809 589 B1**
(45) Date of publication and mention of the grant of the patent: **08.10.2008**
(21) Application number: 05777840.9
(22) Date of filing: 03.07.2005
(51) Int. Cl.: C07C 37/20, C07C 39/16

(54) **A METHOD TO OBTAIN VISUALLY PURE BISPHENOL A**
EIN VERFAHREN ZUR HERSTELLUNG VON OPTISCH REINEM BISPHENOL A
PROCEDE PERMETTANT D'OBTENIR DU BISPHENOL A OPTIQUEMENT PUR

(30) Priority: 19.07.2004 PL 36916604
(43) Date of publication of application: 25.07.2007
(73) Proprietor: PCC Synteza S.A., 47-225 Kedzierzyn-Kozle (PL); Kiedik, Maciej, 47-220 Kedzierzyn-Kozle (PL); Instytut Inzynierii Materialow Polyimerowych i Barwnikow, 87-100 Torun (PL); Sokolowski, Adam, 53-030 Wroclaw (PL)
(72) Inventor: KIEDIK, Maciej, PL-47-220 Kedzierzyn-Kozle (PL); SZYMANSKI, Kazimierz, PL-47-220 Kedzierzyn-Kozle (PL); KOSCIUK, Ryszard, PL-47-200 Kedzierzyn-Kozle (PL); KOLT, Jozef, PL-41-800 Zabrze (PL); RZODECZKO, Anna, PL-47-224 Kedzierzyn-Kozle (PL); KSIAZEK, Krzysztof, PL-44-100 Gliwice (PL); KALEDKOWSKA, Malgorzata, PL-47-220 Kedzierzyn-Kozle (PL); SMOLNIK, Ryszard, PL-47-232 Kedzierzyn-Kozle (PL); MRÓZ, Jerzy, PL-47-220 Kedzierzyn-Kozle (PL)
(74) Representative: Piela, Marek
(86) International application number: PCT/EP2005/053154
(87) International publication number: WO 2006/008230

(56) References cited:
- US-A- 4 517 387
- US-A- 5 315 042
- US-A1- 2004 030 196

## Description

### Technical Field

This invention relates to a method to obtain visually pure Bisphenol A.

### Background Art

The USP Patent No. 5315042 describes a continuous process to manufacture bisphenol A, by contacting phenol with acetone in the presence of an acid catalyst, at increased flowrates through the catalyst, the said flowrates being sufficient to reduce the conversion of acetone whereby the content of acetone is kept at a high level, accounting for a high reaction rate and a suitable increase in the yield of bisphenol A, appropriate separation of the resulting bisphenol A before acetone is depleted. Owing to this, the residence time of bisphenol A in the reaction zone is reduced and the content of byproducts affecting the coloration of bisphenol A is reduced as well. The method has the disadvantage of being too energy consuming, which results from the low conversion of acetone and adequately low (10-13 %) increase in the concentration of bisphenol A in the reaction, whereby the amount of unreacted raw materials for pr ocessing and for regeneration per 1 ton of product is very high.

The International Patent No. WO/19302 of China Petro-Chemical-Corp. describes a method to obtain bisphenol A with high purity, according to which conversion from approximately 4- to approximately 12-fold ratio by mole of phenol to acetone in the presence of a modified ion-exchange resin catalyst is carried out in an essentially vertical, multistage suspension stripping apparatus. The entire phenol required for the reaction of condensation with acetone is fed to the tower-reactor at a point over the top plate, part of the entire acetone required is fed to the plate below the top one, respectively, and to some or all of the plates located at lower levels, whereas an inert gas stream is made to flow upwards through a catalytic chamber, thus forming a liquid-solid suspension, to strip off water from the reaction mixture. Technically, the method is highly complex and impracticable.

The Polish Patent Application P - 347705 describes a process carried out with the use of a double-zone catalytic bed, combined with post-reaction mixture take-off methods that are appropriate for such zones, and with keeping sufficiently high concentrations of water and appropriate temperatures in the catalytic bed, as well as with appropriate purification of the resulting product. Moreover, the process is characterized by low increase [the concentration of] of bisphenol A in the reaction, due to which the consumption of energy in the process is too high.

US 4517387 discloses a process for producing bisphenol A in the presence of three consecutive reactors. No control of concentration of water, bisphenol A or acetone is performed in any of the reactors.

### Disclosure of Invention

### Technical Problem

It was the purpose of the invention to develop a method to manufacture visually pure bisphenol A with selectivities above 95 % so as to obtain an increase in [the concentration of] bisphenol A of at least 23 % in the reaction unit.

### Technical Solution

In the method of the invention, in Step I of the condensation reaction the reactants are pre-reacted to obtain an increase in the concentration of bisphenol A by 2 - 15 %, preferably by 3 - 5 %, while in Step II the reaction is carried out so as to obtain a difference in the concentrations of water between the inlet and outlet of the reactor in the reaction Step II in the range 0.1 - 0.5 %, preferably 0.1 - 0.2 %, while the difference in the concentrations of acetone between the inlet and outlet of the Step II reactor should not exceed 2%, preferably 1 %, and the difference in the concentrations of bisphenol A at the inlet and outlet of the reactor ought to be 1 - 5 %, preferably 1 - 2 %, while the difference in temperatures between the inlet and outlet of the Step II reactor ought not to exceed 15°C, preferably 5°C.

Part of reaction water is removed from the reaction mixture in a continuous manner in the condensation Step II.

The post-reaction mixture resulting from the Step II and containing not less than 15 %, preferably not less than 23 % of bisphenol A, is sent to a distillation system to distill off a fraction that contains acetone, water and phenol, obtaining a residue in the form of crude bisphenol A, containing not more than 5 %, preferably not more than 1 % of phenol, is sent to separation by distillation or to melt fractional crystallization, where visually pure bisphenol A and a residue that contains bisphenol A, its isomers and other byproducts are obtained.

The post-reaction mixture from the condensation reaction Step II is sent to a suspension crystallization phase that proceeds either in one or in two steps, to obtain a crystalline bisphenol A-phenol adduct in the form of a suspension, which is subjected to filtration or centrifugation to separate the adduct which is then fed to a distillation separation system in order to separate bisphenol A. Post-crystallization liquor after water has been distilled off, is fed to the reaction Step II or to a distillation separation phase.

Part of or the entire phenolic stream used in the crystallization (recrystallization) Step II is contacted with an acid ion-exchange resin.

The post-reaction mixture from the condensation reaction Step I, prior to being sent to the reaction Step II, is used for washing the crystalline adduct during filtration or centrifugation after suspension crystallization.

The residue from the distillation separation or from the fractional crystallization is diluted with the stream of phenol and is subjected to static crystallization, to obtain a bisphenol A-phenol adduct, which is sent to the distillation system together with the post-reaction mixture stream from the condensation reaction Step II. The residue from the static crystallization is carried outside the process after phenol has been distilled off.

The residue from the static crystallization is distilled to distill off phenol and is then thermally decomposed in the presence of basic catalysts. The distillate resulting from the distillation is diluted with a stream of phenol and contacted with a cation exchanger, whereas the rearrangement reaction product is sent to the static crystallization.

The residue from the distillation separation or from the melt fractional crystallization is diluted with a stream of phenol and subjected to isomerization by contacting it with an acid ion-exchange resin at a temperature in the range 55 - 95°C, preferably in the range 70 - 85°C.

The isomerization product is fed to the static crystallization phase or the whole or part of it is sent to the distillation system.

The streams containing byproducts and bisphenol A, such as the residue from the distillation separation or from the melt fractional crystallization, are thermally decomposed in the presence of basic catalysts, and the distillate resulting from the decomposition is diluted with a stream of phenol and is then contacted with a macroporous cation exchanger while the rearrangement reaction product is recirculated to the process.

From 25 % to 55 % of the Step I reactor and from 30 % to 95 % of the Step II reactor are filled with the catalyst.

The bottom part of the Step II reactor comprises an ion-exchange resin that does not contain any active promoter, whereas the top of the Step II reactor comprises an ion-exchange resin with an active promoter.

The top of the reactor comprises a layer of beads such that their diameter is from 0.2 to 1.8 mm and which are made of a plastic that is resistant to the reaction environment.

The catalyst used is a mix comprising a cation exchanger with a promoter and a cation exchanger without any active promoter.

The crosslinking degree of the cation exchanger without any active promoter is not more than 4 % DVB while the crosslinking degree of the cation exchanger with a promoter is in the range 2 - 5 % DVB. The crosslinking degree of the cation exchanger without any active promoter is 2 - 5 % DVB while the crosslinking degree of the cation exchanger with a promoter is not more than 4 % DVB.

The catalyst with the promoter is a sulfonated styrene-divinylbenzene copolymer in which 10 % - 99 %, most preferably 50 - 80 %, of sulphonic groups are neutralized by the promoter.

The Step II reactors and, optionally, the Step I reactors are divided each into at least two reaction sections by at least three sets of slot nozzles, located at various heights of the reactors.

From one to six Step II reactors fall to every Step I reactor.

### Advantageous Effects

The method of the invention enables visually pure bisphenol A to be obtained with selectivities up to 98 % and, due to unusually high (20 - 25 %) increase in the concentration of bisphenol A in the reaction, the amount of unreacted raw materials for distillation and processing is much smaller, due to which the consumption of energy in the process is very low.

### Best Mode for Carrying Out the Invention

Example 1.

The condensation process is carried out in two steps: Step I is carried out in a 20-m³ reactor filled with a fixed bed of Purolite CT 122 catalyst with 15 % of sulfonic groups neutralized with 2,2-dimethylthiazolidine. The catalytic bed volume is 6.5 m³.

The reactor is fed upwards with a mixture of phenol and acetone in the ratio of 20 ÷ 1 by mole. The reactor is kept at a temperature in the range 55 - 57°C. The post-reaction mixture from the reactor Step I, containing 5.2 % of bisphenol A and 0.2 % of byproducts, is divided in two equal streams and is sent to two Step II reactors with a capacity of 85 m³ each and containing a fixed bed of 40 m³ of CT 122 cation exchanger with 23 % of sulphonic groups neutralized with 2,2-dimethylthiazolidine. The reactor has 3 sets of filtration-injection slot nozzles in the bottom, middle and top sections. The reaction mixture circulates between the bottom and middle sets of slot nozzles. A stream containing water, acetone and phenol is distilled off from the reaction mixture using an evaporator operated at a 100-mmHg vacuum, at 97°C. The reaction parameters are as follows: temperature is 60°C at the inlet to the reactor and 72°C at the outlet. Water concentration is 1.2 % at the inlet and 1.4 % at the outlet, concentration of acetone is 3.2 % at the inlet and 2.1 % at the outlet, concentration of bisphenol A is 23.1 % at the inlet and 25.4% at the outlet. The post-reaction mixture that contains 25.4 % of bisphenol A and 1.2 % of byproducts is fed to a distillation system to obtain crude bisphenol A, containing 1.2 % of phenol and 4.3 % of byproducts. The crude bisphenol A, of which the purity is approximately 95 %, is distilled at approx. 1 mmHg vacuum, to obtain optical bisphenol A of which the purity is 99.98 % and the color of a molten product is 5 APHA units, and a residue containing a mixture of bisphenol A and by products.

The bisphenol A distillation residue is diluted with a stream of phenol and subjected to static crystallization, whereby an adduct of bisphenol A with phenol comprising: 62 % of bisphenol A, 37.9 % of phenol, 0.1 % of byproducts is obtained. The molten adduct is fed to distillation together with a stream of the post-reaction mixture from the condensation Step II.

The static crystallization residue is subjected to distillation to distill off phenol and the resulting residue is thermally decomposed in the presence of 0.3 % of NaOH, at 240°C, at a vacuum of 10 mmHg. The resulting distillate is diluted with phenol at the ratio of 2 ÷ 1 and is contacted at 72°C with 5 m³ of Purolite CT 175 macroporous cation exchanger. The resulting rearrangement product is recycled to the static crystallization phase.

### Mode for the Invention

Example 2.

A crude bisphenol A, obtained as described in Example 1, is sent to a melt fractional crystallization phase, producing bisphenol A of which the purity is 99.98 % and the color of a molten product is 5 APHA units, and a residue that contains bisphenol A and byproducts, which is sent to thermal decomposition, where at 245°C in the presence of 0.4 % of NaHCO₃, at the vacuum of 15 mmHg, a mixture containing principally phenol, p-isopropenylphenyl, its linear dimers and oligomers is obtained.

The mixture referred to above is diluted with a stream of phenol at the ratio of 2.5 : 1 and is contacted with 5 m³ of Purolite CT 175 macroporous cation exchanger at 75° C. The rearrangement product is fed to distillation along with the post-reaction mixture from the reaction Step II.

Example 3

The melt fractional crystallization residue is diluted with a stream of phenol at the ratio of 1.5 : 1 and is contacted at 78°C with 10 m³ of Purolite CT 175 macroporous cation exchanger to effect isomerization and rearrangement of byproducts, principally the 2,4-isomer, towards bisphenol A. The resulting product is sent to static crystallization and continued to be processed as described in Example 1.

## Claims

1. A method for the manufacture of bisphenol A from phenol and acetone in the presence of an acid ion exchange resin, in two reaction steps, in which the reaction mixture flows in Step II or in the both reaction steps upwards in the reactor, wherein preliminary conversion to obtain an increase in the concentration of bisphenol A in the range 2 - 15 %, preferably 3 - 5 % occurs in Step I of the condensation reaction, while in Step II the reaction is carried out to obtain a difference in the concentrations of water between the inlet and outlet of the reactor in the reaction Step II in the range 0.1 - 0.5 %, preferably 0.1 - 0.2 %, whereas the difference in the concentrations of acetone between the inlet and outlet of the Step II reactor ought not to exceed 2 %, preferably 1 %, the difference between the concentrations of bisphenol A between the inlet and outlet of the reactor ought to be in the range 1 - 5 %, preferably 1 - 2 %, and the difference of temperatures between the inlet and the outlet of the Step II reactor ought not to exceed 15°C, preferably 5°C.

2. A method as claimed in claim 1, wherein part of reaction water is removed in a continuous manner from the reaction mixture in the condensation Step II.

3. A method as claimed in claim 1, wherein the reaction mixture obtained in Step II and containing not less than 15 %, preferably not less than 23 %, of bisphenol A is made to flow to a distillation system to distill off a fraction that contains acetone, water and phenol, obtaining a residue in the form of crude bisphenol A that contains not more than 5 %, preferably not more than 1 %, of phenol is made to flow to a distillation separation phase or to a melt fractional crystallization phase, to obtain therein an optically pure bisphenol A and a residue that contains bisphenol A, its isomers and other byproducts.

4. A method as claimed in claim 1, wherein the post-reaction mixture from the condensation reaction Step II is sent to a suspension crystallization phase to obtain a crystalline adduct of bisphenol A with phenol in the form of a suspension which is then filtered or centrifuged to recover the adduct which is then sent to a distillation separation system to recover bisphenol A and post-crystallization liquor, the latter being sent to the reaction Step II or to a distillation separation phase after water is distilled off therefrom.

5. A method as claimed in claim 4, wherein the resulting adduct is subjected to recrystallization from a phenolic solution, a part or the whole of which is contacted with an acid ion-exchange resin prior to being used in the recrystallization phase, while the purified adduct, after being recovered by filtration or centrifugation, is sent to the distillation separation system in order to recover bisphenol A.

6. A method as claimed in claim 4, wherein the post-reaction mixture from the condensation reaction Step I, prior to being sent to the reaction Step II is used for washing the crystalline adduct during filtration or centrifugation after the suspension crystallization phase.

7. A method as claimed in claim 3, wherein the residue from distillation separation or from fractional crystallization is diluted with a stream of phenol and is subjected to static crystallization, to obtain a bisphenol A-phenol adduct which is sent to the distillation system together with a stream of the post-reaction mixture from the condensation Step II, and a static crystallization residue, which is carried outside the process after phenol is distilled off.

8. A method as claimed in claim 7, wherein phenol, after being distilled off from the static distillation residue is subjected to thermal decomposition in the presence of basic catalysts and the distillate resulting from the separation is diluted with a stream of phenol and is then contacted with a cation exchanger and the rearrangement reaction product is sent to static crystallization.

9. A method as claimed in claim 3, wherein the residue from the distillation separation or from the melt fractional crystallization is diluted with a stream of phenol and is then isomerized by contacting it with an acid ion-exchange resin at a temperature in the range 55 -95°C, preferably in the range 70-85°C.

10. A method as claimed in claim 9, wherein the isomerization product is sent to static crystallization or the whole or part of it is recirculated to the distillation system.

11. A method as claimed in claim 1, wherein the streams that contain byproducts and bisphenol A are thermally decomposed in the presence of basic catalysts and the distillate that results from the decomposition phase is diluted with a stream of phenol prior to being contacted with a macroporous cation exchanger and the rearrangement product is recirculated to the process.

12. A method as claimed in claim 1, wherein from 25 % to 55 % of the Step I reactor and from 30 % to 95 % of the Step II reactors are filled with the catalyst.

13. A method as claimed in claim 1, wherein the bottom of the reactor is filled with an ion-exchange resin that contains no active promoter and the top of the reactor is filled with an ion-exchange resin that contains an active promoter.

14. A method as claimed in claim 1, wherein the top of the reactor is filled with a layer of 0.2-1.8 mm beads made of a plastic material that is resistant to the reaction environment.

15. A method as claimed in claim 1, wherein the catalyst used is a mixture of cation exchangers with and without an active promoter.

16. A method as claimed in claim 1 or in claim 13 or in claim 15, wherein the crosslinking degree of the cation exchanger that contains no active promoter is not more than 4% of DVB and the crosslinking degree of the cation exchanger that contains an active promoter is 2-5% of DVB.

17. A method as claimed in claim 1 or in claim 6 or in claim 14, wherein the crosslinking degree of the cation exchanger that contains no active promoter is in the range 2-5 % of DVB and the crosslinking degree of the cation exchanger that contains an active promoter is not more than 4% of DVB.

18. A method as claimed in claim 15, wherein a sulfonated styrene-divinylbenzene copolymer in which 10 % - 99 %, most preferably 50 - 80 %, of sulfonic groups are neutralized by the promoter is used as the catalyst that contains a promoter.

19. A method as claimed in claim 1, wherein the Step II reactor and, optionally, the Step I reactor are divided into at least two reaction sections each by at least three sets of slot nozzles, located at various levels of the reactor.

20. A method as claimed in claim 1, wherein from one to six reaction Step II reactors fall to a single Step I reactor.

## Patentansprüche

1. Eine Methode für die Herstellung von Bisphenol A aus Phenol und Aceton in Anwesenheit eines Anionenaustauscherharzes, in 2 Reaktionsstufen, bei denen die Reaktionsmischung in die Stufe 2 oder in die beiden Reaktionsstufen den Reaktor aufwärts fließt, in welchem die vorläufige Umwandlung stattfindet, um einen Anstieg in der Konzentration von Bisphenol A im Bereich von 2-15%, vorzugsweise 3-5% in der Stufe 1 zu erreichen, wobei in der Stufe 2 die Reaktion durchgeführt wird, um einen Unterschied zwischen den Wasserkonzentrationen zwischen dem Einlass und dem Auslaß der 2. Reaktorstufe im Bereich von 0,1-0,5%, vorzugsweise 0,1-0,2% zu erreichen, wobei die Differenz zwischen den Acetonkonzentrationen zwischen dem Einlass und dem Auslaß des Reaktors der 2. Stufe 2%, vorzugsweise 1 % nicht überschreiten sollte, die Differenz zwischen den Konzentrationen von Bisphenol A zwischen dem Einlass und dem Auslaß der 2. Stufe des Reaktors sollte im Bereich von 1-5%, vorzugsweise 1-2% sein, und die Temperaturdifferenz zwischen dem Einlass und dem Auslaß der 2. Stufe des Reaktors sollte keine 15°C überschreiten und vorzugsweise 5°C betragen.

2. Eine Methode nach Anspruch 1, wobei ein Teil des Reaktionswassers auf eine kontinuierliche Art und Weise aus der Reaktionsmischung während der Kondensation in der Stufe 2 beseitigt wird.

3. Eine Methode nach Anspruch 1, wobei die Reaktionsmischung, die in der Stufe 2 erzeugt wird und nicht weniger als 15%, vorzugsweise nicht weniger als 23% an Bisphenol A enthält, zum Destillationssystem transportiert wird, um die Fraktion, die Aceton, Wasser und Phenol enthält, herauszudestillieren, wobei ein Rückstand in der Form von rohem Bisphenol A erzeugt wird, welcher nicht mehr als 5%, vorzugsweise nicht mehr als 1 % von Phenol enthält und das dann zu einer Destillationsphase oder zu der Fraktionskristallisationsphase transportiert wird, um optisch pures Bisphenol A und den Rückstand zu gewinnen, der Biosphenol A, seine Isomere und andere Nebenprodukte enthält.

4. Eine Methode nach Anspruch 1, wonach die Mischung nach der Reaktion aus der Kondensationsreaktion der 2. Stufe zu einer Suspensionskristallisationsphase transportiert wird, um einen kristallinen Addukt von Bisphenol A in Form einer Suspension zu gewinnen, der dann filtriert oder zentrifugiert wird, um den Addukt wieder zu gewinnen, der wiederum zum Destillationssystem transportiert wird, um das Bisphenol A und die Lösung nach der Kristallisation wieder zu gewinnen, wobei die Lösung, nachdem das Wasser daraus ausdestilliert worden ist, zur Reaktion der 2. Stufe oder zu einer Destillationsphase geleitet wird.

5. Eine Methode nach Anspruch 4, wonach der entstandene Addukt einer Rekristallisation aus der Phenollösung unterzogen wird, wobei ein Teil der Lösung mit einem Anionenaustauschharz in Kontakt kommt, bevor sie in der Rekristallisationsphase verwendet wird, während der gereinigte Addukt, nachdem er durch Filtrierung oder in einer Zentrifuge wiedergewonnen wird, zum Destillationssystem geleitet wird, um das Bispenol A wieder zu gewinnen.

6. Eine Methode nach Anspruch 4, wonach die Mischung nach der Reaktion aus der Kondensationsreaktion der 1. Stufe, bevor sie zur Reaktion in der 2. Stufen transportiert wird, für das Spülen des kristallinen Addukts während der Filtrierung oder des Zentrifugierens nach der Suspensionskristallisationsphase verwendet wird.

7. Eine Methode nach Anspruch 3, wonach der Rückstand aus der Destillationstrennung oder der Fraktionskristallisation mit einem Phenolstrom verdünnt wird und der statischen Kristallisation unterzogen wird, um den Bisphenol A- Addukt, welcher zum Destillationssystem mit dem Strom der Mischung nach der Reaktion aus der Kondensation in der 2. Stufe transportiert wird, um einen statischen Kristallisationsrückstand zu gewinnen, der aus dem Prozess nach Destillation von Phenol entfernt wird.

8. Eine Methode nach Anspruch 7, wonach das Phenol nach der Destillation aus dem statischen Destillationsrückstand in der Anwesenheit von basischen Katalysatoren thermisch zersetzt wird und das Destillat aus der Trennung mit einem Phenolstrom verdünnt wird und dann mit einem Kationenaustauscher in Kontakt kommt und das Produkt der Reaktion in die statische Kristallisation geleitet wird.

9. Eine Methode nach Anspruch 3, wonach der Rückstand aus der Destillationstrennung oder aus der Fraktionsschmelzkristallisation mit einem Phenolstrom verdünnt wird und durch den Kontakt mit einem Anionenaustauschharz in einer Temperatur von 55-95°C, vorzugsweise in der Temperatur von 70-85°C, isomerisiert wird.

10. Eine Methode nach Anspruch 9, wonach das Produkt der Isomerisierung zur statischen Kristallisation transportiert wird, beziehungsweise das gesamte Produkt oder ein Teil davon in das Destillationssystem wiedereingeführt wird.

11. Eine Methode nach Anspruch 1, wonach die Ströme, die Nebenprodukte und das Bisphenol A enthalten, thermisch in Anwesenheit von basischen Katalysatoren zersetzt werden und das Destillat aus der Zersetzungsphase mit einem Phenolstrom verdünnt wird, bevor es mit einem makroporösen Kationenaustauscher in Kontakt kommt und das Umwandlungsprodukt in den Prozess wiedereingeführt wird.

12. Eine Methode nach Anspruch 1, wonach von 25% bis zu 55% der ersten Reaktorstufe und von 30% bis zu 95% der zweiten Reaktorstufe mit dem Katalysator befüllt werden.

13. Eine Methode nach Anspruch 1, wonach der Boden des Reaktors mit einem Ionenaustauschharz befüllt wird, das keinen Aktivator enthält, und der obere Teil des Reaktors mit einem Ionenaustauschharz befüllt wird, das einen Aktivator enthält.

14. Eine Methode nach Anspruch 1, wonach der obere Teil des Reaktors mit einer Schicht von Kügelchen von 0,2-1,8 mm aus Kunststoff bedeckt wird, der gegen das Reaktionsmedium beständig ist.

15. Eine Methode nach Anspruch 1, wonach der Katalysator eine Mischung aus Kationenaustauschern mit oder ohne Aktivator ist.

16. Eine Methode nach Anspruch 1 oder Anspruch 13 oder Anspruch 15, wonach der Vernetzungsgrad des Kationenaustauschers, der keinen Aktivator enthält, nicht mehr als 4% DVB beträgt, und der Vernetzungsgrad des Kationenaustauschers, der einen Aktivator enthält 2-5% DVB beträgt.

17. Eine Methode nach Anspruch 1 oder 6 oder 14, wonach der Vernetzungsgrad des Kationenaustauschers, der keinen Aktivator enthält, 2-5% DVB beträgt, und der Vernetzungsgrad des Kationenaustauschers, der einen Aktivator enthält, nicht mehr als 4% DVB beträgt.

18. Eine Methode nach Anspruch 15, wonach das sulfonierte Styrol-Divinylbenzen-Copolymer, in welchem 10%-99%, vorzugsweise 50-80% der Sulfongruppen durch den Aktivator neutralisiert sind, als Katalysator, der einen Aktivator enthält, eingesetzt wird.

19. Eine Methode nach Anspruch 1, wonach die 2. Reaktorstufe und, optional, die erste Reaktorstufe in mindestens 2 Reaktionsabschnitte durch mind. 3 Sätze von Düsen, die an verschiedenen Höhen im Reaktor lokalisiert sind, geteilt ist.

20. Eine Methode nach Anspruch 1, wonach von einem bis zu sechs Reaktoren der 2. Stufe in einen Reaktor der 1. Stufe zusammenfließen.

## Revendications

1. Une méthode pour la fabrication de bisphénol A à partir de phénol et d'acétone en présence d'une résine échangeuse d'ion acide, dans une réaction en deux étapes ; dans laquelle le mélange réactionnel s'écoule vers le haut dans le réacteur lors de l'Etape II ou lors des deux étapes ; dans laquelle la conversion préliminaire pour obtenir une augmentation de la concentration de bisphénol A dans la gamme 2 - 15 %, et de préférence 3 - 5 % a lieu lors de l'Etape I de la réaction de condensation, alors que dans l'étape II la réaction est conduite pour obtenir une différence de concentration d'eau entre l'entrée et la sortie du réacteur dans l'Etape II dans la gamme 0.1 - 0.5 %, et de préférence 0.1 - 0.2 %, alors que la différence de concentration d'acétone entre l'entrée et la sortie du réacteur de l'Etape II ne devrait pas dépasser 2 %, et de préférence 1 %, la différence de concentration de bisphénol A entre l'entrée et la sortie du réacteur de l'Etape II devrait être dans la gamme 1 - 5 %, et de préférence 1 - 2 %, et la différence des températures entre l'entrée et la sortie du réacteur de l'Etape II ne devrait pas dépasser 15 °C, et de préférence 5 °C.

2. Une méthode comme revendiquée dans la revendication 1, dans laquelle une partie de l'eau de la réaction est retirée de façon continue du mélange réactionnel dans l'Etape II de condensation.

3. Une méthode comme revendiquée dans la revendication 1, dans laquelle le mélange réactionnel obtenu dans l'étape II et contenant pas moins de 15 %, et de préférence pas moins de 23 %, de bisphénol A est mis à s'écouler un système de distillation pour distiller une fraction qui contient l'acétone, l'eau et le phénol, pour obtenir un résidu de bisphénol A brut contenant pas plus de 5 %, et de préférence pas plus de 1 %, de phénol est mis à s'écouler vers une phase de séparation par distillation ou vers une phase de cristallisation fractionnée, pour obtenir alors du bisphénol A optiquement pur et un résidu qui contient le bisphénol A, ses isomères et d'autres sous-produits.

4. Une méthode comme revendiquée dans la revendication 1, dans laquelle le mélange le mélange post-réactionnel issu de l'Etape II de condensation est envoyer dans une phase de suspension cristallisation pour obtenir un adduit cristallin de bisphénol A avec du phénol sous la forme d'une suspension qui est ensuite filtrée ou centrifugée pour récupérer l'adduit est ensuite envoyé dans un système de séparation par distillation pour récupérer le bisphénol A est une liqueur de post-cristallisation, laquelle est envoyée dans l'Etape II de la réaction ou dans une phase de séparation par distillation après que l'eau soit retirée par distillation.

5. Une méthode comme revendiquée dans la revendication 4, dans laquelle l'adduit obtenu est soumis à une re-cristallisation depuis une solution phénolique, une partie ou la totalité de laquelle est mis en contact avec une résine échangeuse d'ion acide avant d'être utilisée la phase de recristallisation, alors que l'adduit purifié, après être récupéré par filtration ou centrifugation, est envoyé vers un système de séparation par distillation de façon à récupérer le bisphénol A.

6. Une méthode comme revendiquée dans la revendication 4, dans laquelle le mélange post-réactionnel issu de l'Etape I de la réaction de condensation, avant d'être envoyé dans l'Etape II de la réaction est utilisé pour laver l'adduit cristallin pendant la filtration ou la centrifugation après la phase de suspension cristallisation.

7. Une méthode comme revendiquée dans la revendication 3, dans laquelle le résidu de la séparation par distillation ou de la cristallisation fractionnée est dilué avec un écoulement de phénol et est soumis à une cristallisation statique, pour obtenir un adduit bisphénol A-phénol qui est envoyé dans un système de distillation en même temps qu'un flux du mélange post-réactionnel de l'Etape II de condensation, et un résidu de la cristallisation sctatique, qui est entraîné hors du procédé après que le phénol soit éliminé par distillation.

8. Une méthode comme revendiquée dans la revendication 7, dans laquelle le phénol, après être éliminé par distillation du résidu de distillation statique, est soumis à une décomposition thermique en présence de catalyseurs basiques et le distillat résultant de la séparation est dilué par un flux de phénol et est ensuite mis en contact avec échangeur de cation et le produit de la réaction de réarrangement est envoyé en cristallisation statique.

9. Une méthode comme revendiquée dans la revendication 3, dans laquelle le résidu de la séparation par distillation ou de la cristallisation fractionnée est dilué par un flux de phénol et ensuite est isomérisé par mise en contact avec une résine échangeuse d'ion acide à une température dans la gamme 55 - 95 °C, et de préférence dans la gamme 70 - 85 °C.

10. Une méthode comme revendiquée dans la revendication 9, dans laquelle le produit d'isomérisation est envoyé en cristallisation statique ou est mis en recirculation en totalité ou partiellement vers le système de distillation.

11. Une méthode comme revendiquée dans la revendication 1, dans laquelle les flux qui contiennent les sous-produits est le bisphénol A sont décomposés thermiquement en présence de catalyseurs basiques et le distillat qui résulte de la phase de décomposition est dilué dans un flux de phénol avant d'être mis en contact avec un échangeur de cation macroporeux et le produit de réarrangement est mis à recirculer dans le procédé.

12. Une méthode comme revendiquée dans la revendication 1, dans laquelle entre 25 % et 55 % du réacteur de l'Etape I et entre 30 % et 95 % des réacteurs de l'Etape II sont remplis avec le catalyseur.

13. Une méthode comme revendiquée dans la revendication 1, dans laquelle le fond des réacteurs est rempli par une résine échangeuse d'ions qui ne contient pas de promoteurs actifs et le haut du réacteur est rempli avec une résine échangeuse d'ions qui contient un promoteur actif.

14. Une méthode comme revendiquée dans la revendication 1, dans laquelle le haut du réacteur est rempli par une couche de 0.2-1.8 mm de billes faite d'une matière plastique qui est résistante à l'environnement réactionnel.

15. Une méthode comme revendiquée dans la revendication 1, dans laquelle le catalyseur utilisé est un mélange d'échangeurs de cations avec ou sans promoteur actif.

16. Une méthode comme revendiquée dans la revendication 1 ou dans la revendication 13 ou dans la revendication 15, dans laquelle le degré de réticulation de l'échangeur de cations qui ne contient pas de promoteur actif n'est pas supérieur à 4 % de DVB est le degré de réticulation de l'échangeur de cations qui contient un promoteur actif est de 2-5 % de DVB.

17. Une méthode comme revendiquée dans la revendication 1 ou dans la revendication 6 ou dans la revendication 14, dans laquelle le degré de réticulation de l'échangeur de cations qui ne contient pas de promoteur actif est dans la gamme 2-5 % de DVB est le degré de réticulation de l'échangeur de cations qui contient un promoteur n'est pas supérieur à 4 % de DVB.

18. Une méthode comme revendiquée dans la revendication 15, dans laquelle un copolymère sulfonylé styrène-divinylbenzène dans lequel de 10 % à 99 %, est de préférence de 50 à 80 %, des groupes sulfoniques sont neutralisés par le promoteur est utilisé en tant que le catalyseur qui contient un promoteur.

19. Une méthode comme revendiquée dans la revendication 1, dans laquelle le réacteur le l'Etape II et, de façon optionnel, le réacteur de l'Etape I sont divisés en au moins deux sections de réacteur chacun par au moins trois lots de becs, situés à différents niveaux du réacteur.

20. Une méthode comme revendiquée dans la revendication 1, dans laquelle de 1 à 6 réacteurs de l'Etape II rentre dans un seul réacteur de l'Etape 1.
